# EUROPEAN PATENT SPECIFICATION

(11) **EP 2 882 382 B1**
(45) Date of publication and mention of the grant of the patent: **14.04.2021**
(21) Application number: 13827979.9
(22) Date of filing: 08.08.2013
(51) Int. Cl.: A61F 2/95, A61F 2/82, A61M 5/46, A61F 2/848, A61F 2/07

(54) **MEDICAL SECURING DEVICE FOR LIMITING DEPTH OF PENETRATION OF AN ANCHOR WITHIN AN ANATOMY AND A DELIVERY SHEATH**
MEDIZINISCHE BEFESTIGUNGSVORRICHTUNG ZUR BEGRENZUNG DER DURCHDRINGUNGSTIEFE EINES ANKERS IN EINER ANATOMIE UND ZUFÜHRHÜLLE
DISPOSITIF DE FIXATION MÉDICAL POUR LA LIMITATION DE PROFONDEUR DE PÉNÉTRATION D'UN ANCRAGE À L'INTÉRIEUR D'UNE ANATOMIE ET GAINE D'APPORT DE DISPOSITIF MEDICAL

(30) Priority: 10.08.2012 US 201261681677 P; 07.08.2013 US 201313961416
(43) Date of publication of application: 17.06.2015
(73) Proprietor: W. L. Gore & Associates, Inc., Newark, DE 19711 (US)
(72) Inventor: MICHALAK, Christopher, S., Elkton, MD 21921 (US)
(74) Representative: HGF
(86) International application number: PCT/US2013/054049
(87) International publication number: WO 2014/025957

(56) References cited:
- EP-A1- 1 880 693
- EP-A2- 0 896 813
- WO-A1-02/085254
- WO-A2-2004/016201
- WO-A2-2008/033474
- WO-A2-2008/077067
- US-A- 5 843 167
- US-A1- 2003 093 117
- US-A1- 2003 220 683
- US-A1- 2007 093 888
- US-A1- 2008 033 534
- US-A1- 2010 121 373
- US-A1- 2010 274 345
- US-A1- 2010 324 584

## Description

### CROSS REFERENCE TO RELATED APPLICATIONS

This Application claims priority to U.S. Serial No. 61/681,677 filed August 10, 2012.

### BACKGROUND

### Field

The present disclosure generally relates to the field of medicine, and more particularly, to securing devices such as anchors and barbs for securing medical devices within an anatomy or body (e.g., a human body).

### Discussion of the Related Art

A variety of medical devices have been developed for implantation within an anatomy or body (e.g., a human body). Many such devices are implantable within a body lumen (e.g., the vasculature and/or gastrointestinal tract ("GI tract") of a human body). For instance, devices like stents, grafts, and stent-grafts may be implanted within the vasculature and/or GI tract of a human body to reinforce, replace, and/or bridge a damaged, unhealthy, or otherwise diseased portion of a body lumen. These devices may thus, in certain instances, guide blood and/or other fluids through a lumen defined by a cylindrical interior surface. During implantation, however, it is often necessary to anchor such devices in place, so that they will not migrate away from a damaged or diseased portion of the anatomy they are intended to repair.

Although techniques have been developed to hold devices like those described above in place, these techniques may suffer from a variety of shortcomings. For instance, a securing device (such as a medical anchor or barb) may entirely penetrate a body lumen, such that a sharpened portion of the securing device is exposed to (and may damage) surrounding tissue. This may occur, for example, where a securing device comprises a barb intended to penetrate a lumen wall (e.g., the duodenal bulb of the intestinal wall). The barb may, in some circumstances, fully penetrate the lumen wall, which may cause damage to surrounding anatomy and/or open a leakage path into surrounding anatomy.

WO 02/085254 discloses an apparatus for facilitating securement of a vascular graft within a blood vessel, including a shaft dimensioned for passage within a blood vessel and having an expansion member movable between a contracted condition and an expanded condition and a fastener array comprising at least one fastener disposed about a peripheral portion of the expansion member. The fastener array preferably includes a plurality of fasteners, with a fastener deployable into a wall of a blood vessel upon movement of the expansion member to the expanded condition, to thereby engage the vascular graft to secure the vascular graft to a wall of a blood vessel. In one embodiment, the fastener may be a staple with curved staple legs. However this staple does not contain pointed tips.

EP 0 896 813 discloses an anchorage element including a body of shape memory material which, on reaching a transition temperature, passes from a generally retracted position with respect to the associated implant device to an expanded position in which the element anchors the device to the implant site. In one embodiment, the anchorage element may be shaped like the end of a corkscrew. However, such an anchorage element does not contain a depth stop.

US 2003/093117 A1 describes apparatus and methods for partitioning a gastro-intestinal lumen by intraluminally reducing a local cross-sectional area thereof. The apparatus comprises a plurality of anchors adapted for intraluminal penetration into a wall of the gastro-intestinal lumen to prevent migration or dislodgement of the apparatus, and a partition, which may include a drawstring or a toroidal balloon, coupled to the plurality of anchors to provide a local reduction in the cross-sectional area of the gastro-intestinal lumen.

US 5843167 describes an endoprosthesis assembly adapted to be securely positioned within a body lumen. The ass endoprosthesis is provided with one or more hooks adapted to engage tissue that defines the body lumen and includes a resilient self-expanding anchor attached to the tubular graft. The anchor and hooks carried by the anchor are constructed to enable the endoprosthesis to be recaptured in a delivery device to enable repositioning or removal of the endoprosthesis and any time prior to complete deployment of the endoprosthesis in the patient.

US 2010/324584 describes a medical fixation device having a device attachment portion, a compression bearing portion and a barb portion. The barb portion is separated from a device constraining means by the incorporation of the compression bearing portion.

US 2010/121373 describes a removable filter for capturing thrombi in a blood vessel. The filter has primary struts with first ends attached together along a longitudinal axis. Each primary strut has a body member extending from its first end along the longitudinal axis to an anchoring hook. Each primary strut is configured to move relative to the longitudinal axis between an expanded state and a collapsed state. At least one primary strut has a resistant portion next to the anchoring hook. The resistant portion is configured to contact the blood vessel in the expanded state. The filter also has secondary struts. The secondary struts have proximal ends attached together along the longitudinal axis and distal ends located radially from the longitudinal axis in the expanded state. The secondary struts are configured to engage the blood vessel to centralize the filter in the expanded state in the blood vessel.

WO 2008/077067 describes a filter having a first set of members and a second set of members defining a trap sized to fit into a blood vessel. Each of the first and second members are configured to resiliently extend from the trap. At least one of the first set of members includes a first surface for engaging the vessel wall such that the at least one of the first set of members resists downstream movement within the vessel. At least one of the second set of members includes a second surface for engaging the vessel wall such that the at least one second member resists upstream movement within the vessel.

EP0896813 describes an element for anchoring an implant device in place. The anchorage element includes a body made of shape memory material which, on reaching a transition temperature, passes from a generally retracted position with respect to the associated implant device to an expanded position in which the element anchors the device to the implant site.

More suitable techniques for securing a medical device to an intended location are therefore desirable. For instance, a securing device capable of partial implantation in a lumen wall (e.g., such that a lumen wall is not fully punctured) is desirable.

### SUMMARY

Provided herein is a medical device according to claims 1 to 5. The present disclosure includes medical device including a medical securing device comprising, one or more barbs and a delivery sheath that is configured to overlay and compress the medical device for delivery. The medical securing device comprises a barb comprising a pointed tip coupled to a depth stop. The depth sop is configured to limit the depth of penetration of the barb into a body lumen wall. The depth stop comprises an undulating shape or an S-shape comprising an apex portion coupled to a trough portion. The trough portion and the apex portion of the depth stop together, are configured to limit a depth of penetration of the barb into a body lumen wall. The trough portion and the apex portion extend in opposite directions. In use, the medical securing device is configured to be urged into a flat position against an outer surface of the medical device by the delivery sheath and is configured to spring away from the outer surface of the medical device (110, 308) upon release from the delivery sheath, such that the medical securing device (102, 302, 304, 310) is configured to make contact with and penetrate a body lumen wall.

A medical securing device according to the invention may be constructed, in various embodiments, from a length of shape memory wire. Thus, during deployment, the securing device may spring away from the medical device to which the securing device is coupled such that the securing device makes contact with and punctures, to a limited depth, a body lumen wall. The securing device need not, however, fully puncture a body lumen wall. Rather, the depth stop may limit a puncture depth to a depth that does not endanger and/or subject tissue surrounding or external to the lumen wall to damage and/or bleeding or leakage from within the lumen. In various embodiments, a pair of securing devices may be coupled to form an integral two pronged securing device.

### BRIEF DESCRIPTION OF THE DRAWINGS

The features and advantages of the present disclosure will become more apparent from the detailed description set forth below when taken in conjunction with the drawings, wherein:
Figure 1A illustrates a cross-sectional view of a securing device coupled to a medical device;
Figure 1B illustrates a cross-sectional view of a securing device coupled to a medical device and engaged with a body lumen wall;
Figure 2A illustrates a perspective view of a plurality of two pronged securing devices coupled to a medical device; and
Figure 2B illustrates a cross-sectional view of a plurality of securing devices engaged with a body lumen wall.

### DETAILED DESCRIPTION OF THE ILLUSTRATED EMBODIMENTS

Persons skilled in the art will readily appreciate that various aspects of the present disclosure may be realized by any number of methods and apparatuses configured to perform the intended functions. Stated differently, other methods and apparatuses may be incorporated herein to perform the intended functions. It should also be noted that the accompanying figures referred to herein are not all drawn to scale, but may be exaggerated to illustrate various aspects of the present disclosure, and in that regard, the figures should not be construed as limiting. Finally, although the present disclosure may be described in connection with various principles and beliefs, the present disclosure should not be bound by theory.

As used herein, the phrase "securing device" may refer to a device capable of securing a medical device within a human body. In embodiments, a securing device comprises a barb. Also disclosed is a securing device comprising an anchor, a staple, a clip, a hook, a tack, a barb, and the like.

Likewise, as used herein, the phrase "medical device" may refer to a device capable of being secured within a human body. For example, in various embodiments, a medical device may comprise a stent, a graft, a stent-graft, and the like.

While the specific embodiments are described in greater detail below, in general, the present disclosure will focus primarily upon devices for securing a medical device within a body (e.g., a human body). It is also disclosed that these devices may be configured to be applied to treat diseases of the vasculature and/or GI tract, including any disease- where a body lumen is configured to be implanted with a medical device.

In addition, although the devices described herein may focus on application of a medical device to a human body, these devices may be more broadly applied to secure, in use, medical devices within any part of any body (human, mammalian, or otherwise). Moreover, although the disclosure provided herein may focus, in part, upon disclosures in which a medical device is configured to be secured to a body lumen, the devices described herein may apply equally to tissue to tissue fixation as well as to fixation of medical devices to non-luminal body tissue.

In various embodiments, a medical device including a medical securing device (e.g., a securing device comprising one or more anchors or barbs) and a delivery sheath that is configured to overlay and compress the medical device for delivery is disclosed. A medical securing device comprises a barb comprising a pointed tip coupled to a depth stop, and a depth stop, in turn, comprises an undulating shape or an S-shape comprising an apex portion coupled to a trough portion, wherein the trough portion and the apex portion of the depth stop together, are configured to limit a depth of penetration of the barb into a body lumen wall. A barb may be constructed, in various embodiments, from a length of shape memory wire. Thus, in use, the medical securing device is configured to be urged into a flat position against an outer surface of the medical device by the delivery sheath and, during deployment, upon release from the delivery sheath, the medical securing device is configured to spring away from the outer surface of the medical device to which the medical securing device is coupled, such that the medical securing device is configured to make contact with and puncture, to a limited depth, a body lumen wall. A medical securing device may not, however, fully puncture a body lumen wall. Rather, the depth stop may limit a puncture depth to a depth that does not endanger and/or subject tissue surrounding or external to the lumen wall to damage and/or bleeding or leakage from within the lumen.

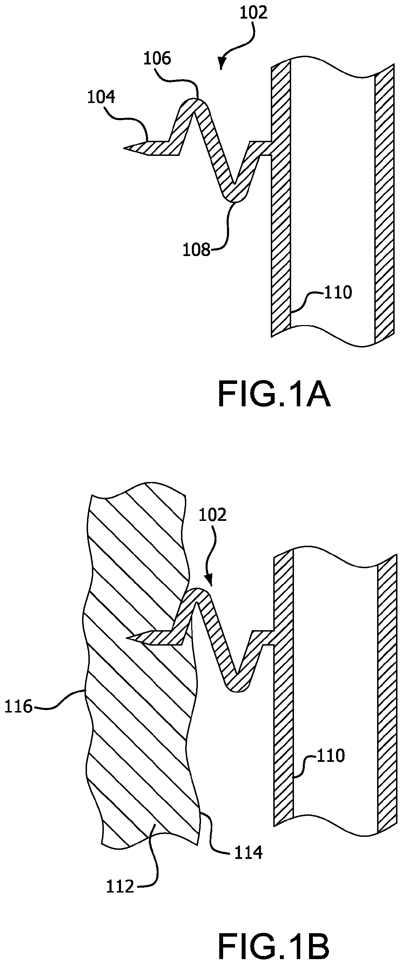

With reference now to Figure 1A, a cross-sectional view of a securing device 102 is shown. A securing device 102 comprises a depth stop, which may limit a depth of penetration of the device 102 into a body lumen. A securing device 102 further comprises a pointed tip 104, which is coupled to a depth stop. The depth stop comprises an apex or ridge portion 106 coupled to a trough or depression portion 108. Thus, a depth stop comprises an undulating shape, or an "S". A medical securing device 102 is coupled to a medical device (e.g., a stent or stent-graft) 110, as shown.

With reference now to Figure 1B, a cross-sectional view of a medical securing device 102 penetrating a body lumen wall 112 is shown. As depicted, a medical securing device 102 may secure and/or stabilize a medical device 110 as the medical device 110 engages a body lumen wall 112. Further, a depth stop of a securing device 102, as described above, may limit a depth of penetration of the securing device 102 into the body lumen wall 112. More particularly, in various embodiments, a ridge portion 106 and/or a trough portion 108 of a depth stop may, together, limit a depth of penetration of a securing device 102 into a body lumen wall 112. For example, a medical securing device 102 may encounter resistance as the ridge portion 106 and the trough portion 108 engage or make contact with a luminal surface 114 of the body lumen wall 112 sufficient to halt further penetration of the securing device 102 into the body lumen wall 112. Thus, the undulating shape of a depth stop, as shown, prevents a securing device 102 from fully puncturing a body lumen wall 112. Rather, the progress of the pointed tip 104 of a securing device 102 may be halted by a depth stop in its progress through a body lumen partway through the body lumen.

Accordingly, in various embodiments, a pointed tip 104 of a medical securing device 102 may not puncture or rupture an abluminal surface 116 of a body lumen wall 112. This may, in turn, and as discussed above, prevent fluid leakage through a lumen wall 112 as well as tissue damage to tissue external to abluminal surface 116. Additionally, a medical securing device 102 may be constructed to a specific dimension or size (e.g., a pointed tip 104 may be constructed to a particular length prior to placement of a depth stop) depending upon a thickness of a body lumen wall 112 into which the medical securing device 102 must penetrate. In other words, a medical securing device 102 may be designed to fit and/or be used with a variety of body lumens (each body lumen potentially having a unique thickness).

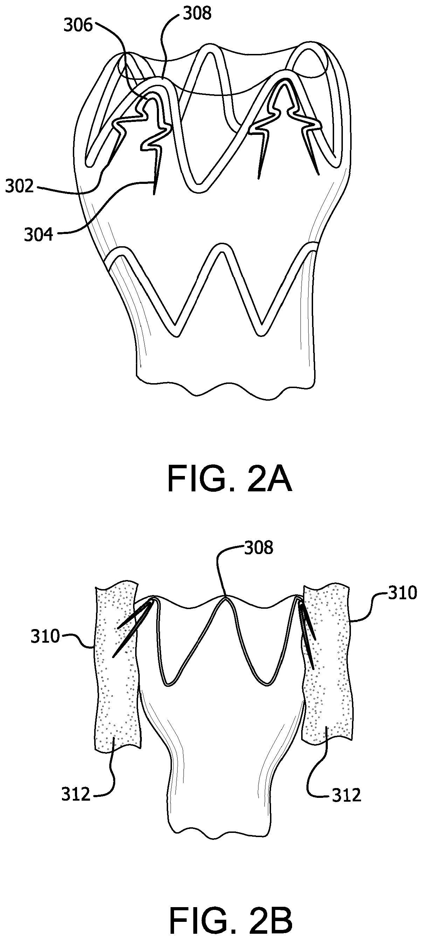

With reference to Figure 2A, a perspective view of a two pronged device is shown. As illustrated, a two pronged device may comprise a plurality of medical securing devices 302 and 304. Each medical securing device 302 and 304 may comprise, as described above, a depth stop. Moreover, in various embodiments, a medical securing device in a pair of medical securing devices 302 and 304 may be coupled to the other medical securing device in the pair of securing devices, such that the pair of medical securing devices 302 and 304 comprises an integral two pronged device. A two pronged device may, as shown, be symmetrical about a centerline defined by a depression or trough 306 formed by the intersection of the medical securing devices 302 and 304. In various embodiments, a two pronged device may be formed from a length of wire, e.g., a length of shape memory wire such as a length of wire comprising Nickel Titanium alloy (or NiTi).

A medical securing device, which may be a two pronged device (hereinafter simply a "medical securing device" or "medical securing devices" for ease of reference), is coupled to a medical device 308. Similarly, in various embodiments, a plurality of medical securing devices may be coupled to a medical device 308 and/or a plurality of medical devices. A medical securing device may be coupled to a medical device 308 by way of any method known in the art (e.g., chemical adhesion, thermal adhesion, metallurgical adhesion or bonding, integral construction with the medical device, and the like).

Referring to Figure 2B, one or more medical securing devices (e.g., one or more two pronged devices) 310 are coupled to a medical device 308 and are configured to be deployed within a body lumen such that the medical securing devices 310 engage a body lumen wall 312. A medical securing device may, in use, be configured to lay flat or substantially flat against a medical device 308 surface (e.g., as illustrated at Figure 2A) until the medical device is positioned as desired (e.g., by a physician manipulating the medical device and/or a delivery lumen containing the medical device) within a body lumen. A medical securing device 310 may, is urged into a flat position against a medical device 308 surface by a delivery sheath, which may overlay and, to some extent, compress the securing device 310 and/or the medical device 308 for delivery. A medical securing device 310 thus compressed may be spring loaded. Similarly, in various embodiments, a securing device 310 may adopt a flattened profile in response to a particular ambient temperature (e.g., the securing device 310 may comprise a heat sensitive shape memory alloy, which may flatten or adopt a disengaged profile during a martensite phase).

A medical securing device 310 may be configured to be deployed with a medical device delivery system (e.g., through the working channel of a therapeutic endoscope having, for example, a diameter and/or radius less than or equal to about six millimeters, and using an everting sleeve delivery system). During deployment, one or more medical securing devices 310 may spring out, away from a medical device 308, such that each medical securing device 310 makes contact with and penetrates a body lumen wall 312. A medical securing device 310 may deploy in response to removal of a delivery sheath, as described above. Where a medical securing device 310 springs into a deployed position, the medical securing device 310 may do so in response to a spring loaded force, such as a natural tendency of the medical securing device 310 (which, again, may comprise a shape memory material), to return to a formed shape or configuration. Similarly, a medical securing device 310 may deploy in response to being heated to a particular temperature (e.g., a typical normothermic human body temperature, which may cause the securing device 310 to enter an austenite phase). Further disclosed, a medical securing device 310 may make contact with a body lumen wall 312 at a variety of angles, including acute angles, a ninety degree angle, and obtuse angles.

Further disclosed, a medical securing device 310 is configured to penetrate, as discussed above, a body lumen wall 312 to a depth that is limited by a depth stop. Thus, a medical device may be configured to be deployed within a body lumen, such that the medical securing device is unable to penetrate an outer surface of a body lumen wall 312. This feature may, as described herein, protect tissue external to a body lumen wall 312 from damage by a securing device 310. This feature may also prevent a puncture or rupture of a lumen wall 312, which might result in fluid leakage between the body lumen and tissue external to the lumen.

Also disclosed herein is a medical securing device comprising a threaded or threadable structure. Similarly, in various embodiments, a delivery lumen may comprise a threaded or threadable structure. For example, where a medical securing device comprises a threaded structure, the medical securing device may rotate through a threaded delivery lumen and/or deploy within a body lumen and/or body tissue in a rotating manner. Simply put, it is disclosed that a medical securing device may be deployed like a screw. A medical securing device thus deployed may incise or cut a spiraling channel within body tissue, which may aid in the secure placement of the device within the tissue.

The medical securing devices described herein include or incorporate one or more barbs or hooks. For example, a medical securing device (including a barb, as discussed above) includes one or more barbs, each of which may have a pointed tip that points in a distal direction. Thus, a barbed securing device may be easily deployed within tissue but resist motion in a distal direction).

Further disclosed herein, a plurality of medical securing devices may be loaded into a delivery lumen for sequential delivery within a body. These devices may be loaded within a delivery lumen in a straightened configuration and/or a substantially straightened configuration, which may facilitate delivery to body tissue in a minimally biologically invasive manner.

With brief regard to grafts and stent-grafts, many graft materials are known, and in various embodiments, these materials can be used in combination and assembled together to comprise a graft. These materials may be further extruded, coated and/or formed from wrapped films, and/or a combination thereof. Polymeric materials, biodegradable materials, and/or natural materials can be used for specific applications.

In various embodiments, a graft may comprise synthetic polymers including nylon, polyacrylamide, polycarbonate, polyformaldehyde, polymethylmethacrylate, polytetrafluoroethylene, polytrifluorochlorethylene, polyvinylchloride, polyurethane, elastomeric organosilicon polymers, polyethylene, polypropylene, polyurethane, polyglycolic acid, polyesters, polyamides, their mixtures, blends, and copolymers. In a variety of embodiments, a graft may be made from a class of polyesters such as polyethylene terephthalate including DACRON® and MYLAR® and polyaramids such as KEVLAR®, polyfluorocarbons such as polytetrafluoroethylene (PTFE) with and without copolymerized hexafluoropropylene (TEFLON® or GORE-TEX®), and porous or nonporous polyurethanes. Further, in a variety of embodiments, a graft may comprise expanded fluorocarbon polymers (especially PTFE).

In various embodiments, fluoropolymers may include polytetrafluoroethylene (PTFE), expanded PTFE (ePTFE), fluorinated ethylene propylene (FEP), copolymers of tetrafluoroethylene (TFE) and perfluoro (propyl vinyl ether) (PEA), homopolymers of polychlorotrifluoroethylene (PCTFE), and its copolymers with TFE, ethylene-chlorotrifluoroethylene (ECTFE), copolymers of ethylene-tetrafluoroethylene (ETFE), polyvinylidene fluoride (PVDF), and polyvinylfluoride (PVF). In various embodiments, a graft may comprise any combination of the materials listed above. Further, in various embodiments, a graft may be substantially impermeable and/or permeable to bodily fluids. A substantially impermeable graft may be made from materials that are substantially impermeable to bodily fluids or can be constructed from permeable materials treated or manufactured to be substantially impermeable to bodily fluids (e.g. by layering different types of materials described above or known in the art). In various embodiments, a medical device, as described above, may be made from any combination of the materials described above, including ePTFE.

Any stent may be generally cylindrical when restrained and/or when unrestrained and may comprise helically arranged undulations having a plurality of helical turns. In a variety of embodiments, undulations may be aligned so that they are "in-phase" with each other. More specifically, undulations may comprise apices in opposing first and second directions. When these undulations are in-phase, apices in adjacent helical turns are aligned so that apices can be displaced into respective apices of a corresponding undulation in an adjacent helical turn. In certain embodiments, undulations may have a sinusoidal shape, a U shape, a V shape, and/or an ovaloid shape.

In various embodiments, a stent may be fabricated from a variety of biocompatible materials including commonly known materials (or combinations of materials) used in the manufacture of implantable medical devices. Such materials may include 316L stainless steel, cobalt-chromium-nickel-molybdenum-iron alloy ("cobalt- chromium"), other cobalt alloys such as L605, tantalum, nitinol, or other biocompatible metals. In some embodiments, any stent and/or stent-graft described herein may comprise a balloon expandable stent and/or stent-graft and/or a self-expanding stent and/or stent- graft. Further, in certain embodiments, a stent may comprise a wire wound stent, which may or may not comprise undulations.

## Claims

1. A medical device (110, 308) including a medical securing device (102, 302, 304, 310) and a delivery sheath that is configured to overlay and compress the medical device (110, 308) for delivery, the medical securing device (102, 302, 304, 310) comprising:
a barb comprising a pointed tip (104) coupled to a depth stop (106, 108), the depth stop (106, 108) configured to limit a depth of penetration of the barb into a body lumen wall (112, 312) wherein the depth stop (106, 108) comprises a undulating shape or an S-shape comprising a trough portion (108, 306) coupled to an apex portion (106), wherein the trough portion (108) and the apex portion (106) of the depth stop together, are configured to limit a depth of penetration of the barb into a body lumen wall (112), and
wherein the trough portion (108, 306) and the apex portion (106) extend in opposite directions, and
wherein, in use, the medical securing device (102, 302, 304, 310) is configured to be urged into a flat position against an outer surface of the medical device (110, 308) by the delivery sheath and is configured to spring away from the outer surface of the medical device (110, 308) upon release from the delivery sheath, such that the medical securing device (102, 302, 304, 310) is configured to make contact with and penetrate a body lumen wall.

2. The medical device (110, 308) of claim 1 wherein the barb is constructed from a length of shape memory wire.

3. The medical device (110, 308) of claim 1, wherein the trough portion (108, 306) is coupled to one of: a stent or a stent graft (110).

4. The medical device (110, 308) of claim 1, wherein the barb is coupled to one of: stent or a stent graft (110).

5. The medical device (110, 308) of claim 1, wherein the depth stop (106, 108) comprises an "S" shape.

## Patentansprüche

1. Medizinische Vorrichtung (110, 308), die eine medizinische Sicherungsvorrichtung (102, 302, 304, 310) und eine Einführhülse umfasst, die so gestaltet ist, dass sie die medizinische Vorrichtung (110, 308) überlagert und zur Einführung komprimiert, wobei die medizinische Sicherungsvorrichtung (102, 302, 304, 310) folgendes umfasst:
einen Stachel, der eine zulaufende Spitze (104) umfasst, die mit einem Tiefenanschlag (106, 108) gekoppelt ist, wobei der Tiefenanschlag (106, 108) so gestaltet ist, dass er eine Penetrationstiefe des Stachels in eine Körperlumenwand (112, 312) begrenzt, wobei der Tiefenanschlag (106, 108) eine Wellenform oder eine S-Form umfasst, die einen Senkenteil (108, 306) umfasst, der mit einen Gipfelteil (106) gekoppelt ist, wobei der Senkenteil (108) und der Gipfelteil (106) des Tiefenanschlags gemeinsam so gestaltet sind, dass sie eine Penetrationstiefe des Stachels in eine Körperlumenwand (112) begrenzen, und
wobei sich der Senkenteil (108, 306) und der Gipfelteil (106) in entgegengesetzte Richtungen erstrecken, und
wobei die die medizinische Sicherungsvorrichtung (102, 302, 304, 310) im Einsatz so gestaltet ist, dass sie durch die Einführhülse an eine flache Position an einer äußeren Oberfläche der medizinischen Vorrichtung (110, 308) geführt wird, und wobei sie so gestaltet ist, dass sie von der äußeren Oberfläche der medizinischen Vorrichtung (110, 308) weg federt, wenn sie von der Einführhülse gelöst wird, so dass die medizinische Sicherungsvorrichtung (102, 302, 304, 310) so gestaltet ist, dass sie Kontakt mit einer Körperlumenwand herstellt und in diese eindringt.

2. Medizinische Vorrichtung (110, 308) nach Anspruch 1, wobei der Stachel aus einer Länge Formgedächtnisdraht gestaltet ist.

3. Medizinische Vorrichtung (110, 308) nach Anspruch 1, wobei der Senkenteil (108, 306) mit einem der folgenden gekoppelt ist: einem Stent oder einem Stent-Graft (110).

4. Medizinische Vorrichtung (110, 308) nach Anspruch 1, wobei der Stachel mit einem der folgenden gekoppelt ist: einem Stent oder einem Stent-Graft (110).

5. Medizinische Vorrichtung (110, 308) nach Anspruch 1, wobei der Tiefenanschlag (106, 108) eine "S"-Form umfasst.

## Revendications

1. Dispositif médical (110, 308) comprenant un dispositif de fixation médical (102, 302, 304, 310) et une gaine de mise en place qui est conçue pour recouvrir et comprimer le dispositif médical (110, 308) pour la mise en place, le dispositif de fixation médical (102, 302, 304, 310) comprenant :
un ardillon comprenant une pointe pointue (104) accouplé à une butée de profondeur (106, 108), la butée de profondeur (106, 108) étant conçue pour limiter une profondeur de pénétration de l'ardillon dans une paroi de lumière corporelle (112, 312), la butée de profondeur (106, 108) comprenant une forme ondulée ou une forme en S comprenant une partie en creux (108, 306) accouplée à une partie de sommet (106), la partie en creux (108) et la partie de sommet (106) de la butée de profondeur étant conçues ensemble pour limiter une profondeur de pénétration de l'ardillon dans une paroi de lumière corporelle (112), et
la partie en creux (108, 306) et la partie de sommet (106) s'étendant dans des directions opposées, et
lors de l'utilisation, le dispositif de fixation médical (102, 302, 304, 310) étant conçu pour être poussé dans une position plate contre une surface extérieure du dispositif médical (110, 308) par la gaine de mise en place et étant conçu pour s'écarter par ressort de la surface extérieure du dispositif médical (110, 308) lors de la libération de la gaine de mise en place, de sorte que le dispositif de fixation médical (102, 302, 304, 310) soit conçu pour entrer en contact avec et pénétrer dans une paroi de lumière corporelle.

2. Dispositif médical (110, 308) selon la revendication 1, l'ardillon étant construit à partir d'une longueur de fil à mémoire de forme.

3. Dispositif médical (110, 308) selon la revendication 1, la partie en creux (108, 306) étant accouplé à : un stent ou une endoprothèse (110).

4. Dispositif médical (110, 308) selon la revendication 1, l'ardillon étant accouplé à : un stent ou une endoprothèse (110).

5. Dispositif médical (110, 308) selon la revendication 1, la butée de profondeur (106, 108) comprenant une forme en « S ».
